# EUROPEAN PATENT SPECIFICATION

(11) **EP 4 032 973 B1**
(45) Date of publication and mention of the grant of the patent: **19.07.2023**
(21) Application number: 21152684.3
(22) Date of filing: 21.01.2021
(51) Int. Cl.: C12N 5/0783, A61K 35/17

(54) **MODIFIED T CELLS FOR ADOPTIVE IMMUNOTHERAPY**
MODIFIZIERTE T-ZELLEN FÜR DIE ADOPTIVE IMMUNTHERAPIE
LYMPHOCYTES T MODIFIÉS POUR L'IMMUNOTHÉRAPIE ADOPTIVE

(43) Date of publication of application: 27.07.2022
(73) Proprietor: Guizhou Sinorda Biotechnology CO., Ltd., Guiyang, Guizhou 550008 (CN)
(72) Inventor: YANG, Yuan, Guiyang, Guizhou 550008 (CN); DU, Hang, Guiyang, Guizhou 550008 (CN); TANG, Jingling, Guiyang, Guizhou 550008 (CN); HU, Pingsheng, Guiyang, Guizhou 550008 (CN)
(74) Representative: Zacco Sweden AB

(56) References cited:
- US-A1- 2018 318 419
- PILLSBURY CLAIRE ET AL: "Abstract 4151: Siglec-15 is a novel immunomodulatory effector of adaptive immune evasion in acute lymphoblastic leukemia", CLINICAL RESEARCH (EXCLUDING CLINICAL TRIALS), 1 July 2019 (2019-07-01), pages 4151-4151, XP055819476, DOI: 10.1158/1538-7445.AM2019-4151 Retrieved from the Internet: URL:http://dx.doi.org/10.1158/1538-7445.AM 2019-4151>
- WANG JUN ET AL: "Siglec-15 as an immune suppressor and potential target for normalization cancer immunotherapy", NATURE MEDICINE, NATURE PUB. CO, NEW YORK, vol. 25, no. 4, 4 March 2019 (2019-03-04), pages 656-666, XP036749914, ISSN: 1078-8956, DOI: 10.1038/S41591-019-0374-X [retrieved on 2019-03-04]
- ANGATA TAKASHI: "Abstract", JOURNAL OF BIOMEDICAL SCIENCE, vol. 27, no. 1, 3 January 2020 (2020-01-03), XP055786639, DOI: 10.1186/s12929-019-0610-1 Retrieved from the Internet: URL:http://link.springer.com/article/10.11 86/s12929-019-0610-1/fulltext.html>
- CAO GUANGCHAO ET AL: "Normalization cancer immunotherapy: blocking Siglec-15!", SIGNAL TRANSDUCTION AND TARGETED THERAPY, vol. 4, no. 1, 19 April 2019 (2019-04-19), XP055819474, DOI: 10.1038/s41392-019-0045-x Retrieved from the Internet: URL:http://www.nature.com/articles/s41392- 019-0045-x>
- XIUBAO REN: "Immunosuppressive checkpoint Siglec-15: a vital new piece of the cancer immunotherapy jigsaw puzzle", CANCER BIOLOGY & MEDICINE, vol. 16, no. 2, 1 January 2019 (2019-01-01), page 205, XP055819473, CN ISSN: 2095-3941, DOI: 10.20892/j.issn.2095-3941.2018.0141
- SHIV PILLAI ET AL: "Siglecs and Immune Regulation", ANNUAL REVIEW OF IMMUNOLOGY, vol. 30, no. 1, 23 April 2012 (2012-04-23) , pages 357-392, XP055289983, ISSN: 0732-0582, DOI: 10.1146/annurev-immunol-020711-075018
- SUN JINGWEI ET AL: "Siglec-15 as an Emerging Target for Next-generation Cancer Immunotherapy", CLINICAL CANCER RESEARCH, vol. 27, no. 3, 1 February 2021 (2021-02-01), pages 680-688, XP055819472, US ISSN: 1078-0432, DOI: 10.1158/1078-0432.CCR-19-2925 Retrieved from the Internet: URL:https://clincancerres.aacrjournals.org /content/clincanres/27/3/680.full.pdf?casa _token=HxjBse3-URgAAAAA:uOevsEp132fJUA2dmv JeBOQyrkHN4eoWqKTc4TQENfHrCBICYTrODnVw6n0L 4TwOXyWFNNyYIbE>

## Description

### Field of the invention

The present invention relates to the field of therapeutic treatment. In particular, it relates to cancer therapy based on administration of autologous cells to a patient in need thereof, to cells useful in such therapy, and to methods for preparing such cells.

### Background

Adoptive immunotherapy, using autologous, *in vitro* expanded lymphocytes isolated from a tumor-draining sentinel lymph node is known in the art (1). Adoptive immunotherapy, including collection and expansion of autologous tumor reactive lymphocytes with retransfusion to the patient, has also been explored in malignant melanoma (2).

WO2018/234516 teaches a method for expanding anti-tumor T-cells, together with a phagocytosable particle, having one or more tumor neoantigenic constructs tightly associated thereto.

Adoptive T cell transfer therapy, in which autologous or allogenic T cells are infused into patients with cancer, has shown considerable promise in recent years (3).

Siglecs are vertebrate cell-surface receptors that recognize sialylated glycans. SIGLEC15 is a type-I transmembrane protein consisting of: (i) two immunoglobulin (Ig)-like domains, (ii) a transmembrane domain containing a lysine residue, and (iii) a short cytoplasmic tail. SIGLEC15 is expressed on macrophages and/or dendritic cells of human spleen and lymph nodes (4). SIGLEC15 messenger RNA expression is minimal in most normal human tissues and various immune cell subsets but can be found in macrophages, most in M2 macrophages (4). SIGLEC15 can not only regulate osteoclast differentiation, but also suppresses T cell responses (5).

It has been suggested in US2019/202912 to use antibodies binding to SIGLEC15 in cancer therapy.

### Summary of the invention

The present inventors have surprisingly found that downregulation of *SIGLEC15* in T cells derived from sentinel lymph nodes can mitigate the immune compromise rendered by cancer-derived immune suppressive factors, thus mitigating the tumor's ability to invade and metastasize.

Thus, in a first aspect, the present invention relates to a method for obtaining a population of T-cells having reduced expression of *SIGLEC15,* comprising
S1'. Providing a T cell containing lymph node tissue removed from a subject, said lymph node tissue being obtained from a lymph node identified as a lymph node draining lymphatic fluid from a cancerous tumor in said subject;
S2. Extracting cells from the identified lymph node;
S3. Reducing expression of *SIGLEC15* in the extracted cells; and
S4. Expanding the extracted cells with reduced expression of *SIGLEC15* under conditions favouring T cell expansion.

The expression of *SIGLEC15* is reduced by downregulation of expression of a gene encoding SIGLEC15 by siRNA transfection, shRNA transfection, or CR!SPR-mediated gene editing.

In some embodiments, the expression of *SIGLEC15* is reduced by downregulation of expression of a gene encoding SIGLEC15 by siRNA transfection using an siRNA molecule having a nucleotide sequence according to SEQ ID NO: 1, SEQ ID NO: 2, SEQ ID NO: 3, SEQ ID NO: 4, SEQ ID NO: 5, or SEQ ID NO: 6.

In some embodiments, the conditions favouring T cell expansion comprise maintenance of the cells in the presence of interleukin-2.

In a further aspect, the present invention relates to a population of T cells with reduced expression of *SIGLEC15* obtainable by the method according to the invention for use in medicine.

In a further aspect, the present invention relates to a population of T cells according to the invention for use in a method for treatment of a cancer.

In some embodiments, the cancer is a solid tumor.

In some embodiments, the cancer is the cancerous tumor of the subject from which the T cells originates, or a metastasis thereof.

In some embodiments, the cancer is selected from the group of colorectal cancer, malignant melanoma, cervical carcinoma, Head & Neck Squamous Cell Carcinoma (HNSCC), Non-Small Cell Lung Carcinoma (NSCLC).

Also disclosed is the use of a population of T cells according to the invention in the preparation of a pharmaceutical composition for use in a method of treatment according to the invention.

In a further aspect, the present invention relates to a pharmaceutical composition comprising a population of T cells according to the invention, and optionally pharmaceutically acceptable excipients and or carriers.

### Brief description of the drawings

Figure 1: A flow-chart illustrating a method for obtaining a population of T cells according to the present invention.
Figure 2: A flow-chart illustrating a method of treatment according to the present invention.
Figure 3: A: Relative expression of *SIGLEC15* in sentinel and non-sentinel lymph nodes, respectively. B: Relative expression of *SIGLEC15* in sentinel lymph nodes before and after siRNA knock-down
Figure 4: Protein expression of SIGLEC15 in different cell types. A: Comparison between Sentinel Nodes (SN) and Non-Sentinel Nodes (NSN) across cell types; B: Comparison across cell types for Sentinel Nodes (SN).
Figure 5: T cell functional cytokine release after SIGLEC15 knock-down.

### Detailed description of the invention

Lymph nodes draining the primary tumor are essential for the initiation of an effective anti-tumor T-cell immune response. However, cancer-derived immune suppressive factors render the sentinel lymph nodes(SN) immune compromised, enabling tumors to invade and metastasize.

The present inventors have studied different mechanisms underlying this immune escape to devise therapeutic intervention strategies to halt tumor spread in early clinical stages. The present invention thus draws on an understanding of microenvironment regulations on transcription level in lymph nodes of cancer patients, such as colorectal cancer patients.

Several cytokines limit tumor cell growth by a direct anti-proliferative or pro-apoptotic activity, or indirectly by stimulating the cytotoxic activity of immune cells against tumor cells. Interleukin-2 (IL-2) 2 is viewed as a key cytokine in promoting the expansion of natural killer (NK) cells and T lymphocytes. NK cells and T cells are the primary lymphocyte subsets that kill tumors. Tumor Necrosis Factor α (TNF-α) is mainly considered as a mediator of anti-tumour immune responses. Interferon γ (IFN-γ) is a pleiotropic molecule with associated anti-proliferative, pro-apoptotic and antitumor mechanisms. Release of IFN-y is e.g. a key potency indicator in the quality assessment of the CAR-T cell product tisagenlecleucel (6).

It has been found that T cells that have been isolated from tumor-draining lymph nodes and treated to reduce expression of SIGLEC15 have an improved anti-tumor effect, as assessed by release profile of the effector cytokines discussed above. As disclosed in the experimental section below, T cells with reduced SIGLEC15 expression show an increased release of the cytokines IL-2, TNF-α, and IFN-γ.

The present invention consequently relates in part to the use of autologous T cells that have been treated to reduce expression of SIGLEC15 in cancer therapy, and to corresponding methods of treatment. The invention also relates to methods for preparing and obtaining such T cells, to T cells obtained or obtainable by such methods, and to pharmaceutical compositions comprising such T cells.

Thus, in a first aspect, the present invention relates to a method that does not involve any surgical step performed on a human or animal body, which method for obtaining a population of T-cells having reduced expression of *SIGLEC15,* comprises
S1'. Providing a T cell containing lymph node tissue removed from a subject, said lymph node tissue being obtained from a lymph node identified as a lymph node draining lymphatic fluid from a cancerous tumor in said subject;
S2. Extracting cells from the identified lymph node;
S3. Reducing expression of *SIGLEC15* in the extracted cells; and
S4. Expanding the extracted cells with reduced expression of *SIGLEC15* under conditions favouring T cell expansion.

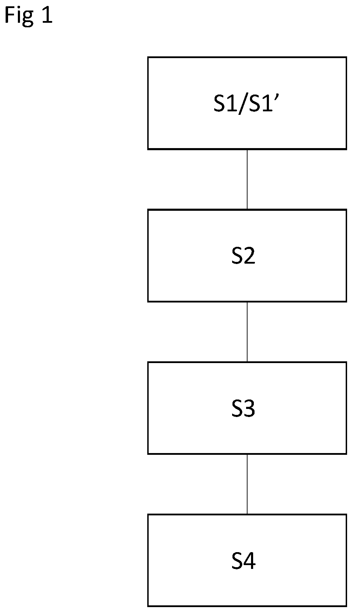

The above method is schematically illustrated in Figure 1.

Identification of a lymph node as a lymph node draining lymphatic fluid from a cancerous tumor can be done as known in the art, e.g. as described by Dahl and co-workers (7). In brief, a detectable and physiologically acceptable dye is injected in or around the tumor. The lymphatic fluid carries the dye from the injection site to a draining lymph node which is thus stained by the dye and identified as a draining lymph node. Exemplary dyes are patent blue, Evans blue, and Alexa Fluor^{®} 488 dye.

Extraction of cells from the lymph node identified as a draining lymph node can be done in various ways. The entire lymph node may be removed from the patient by excision. It is also possible to only obtain a piece of lymph node tissue, e.g. through a biopsy. Extracted cells of the lymph node tissue may be made into single-cell suspension as known in the art (8).

The expression of SIGLEC15 in the extracted cells is then reduced.

In some embodiments, the expression of *SIGLEC15* is reduced by downregulation of expression of a gene encoding SIGLEC15 by siRNA transfection. Briefly, transfection of lymph node single cell with siRNA may be performed. 1 µmol/L siRNA may be transfected in a 96-well tissue culture plate with Accell siRNA delivery media (GE Dharmacon) for 72 hours according to the manufacturer's instruction. RT-PCR is normally used to assess SIGLEC15 mRNA expression in the siRNA transfected and non-transfected groups. Other protocols are well known in the art (9) and will not be described in detail here. Protocols and reagents for downregulating expression of a specific gene by siRNA are also commercially available from a number of companies, including without prejudice ThermoFisher Scientific, Sigma-Aldrich, Qiagen, and GE Dharmacon.

In some embodiments, the expression of *SIGLEC15* is reduced by downregulation of expression of a gene encoding *SIGLEC15* by Short Hairpin RNA (shRNA) transfection. Such protocols are well known in the art (10) and will not be described in detail here. In brief, pGPU6 vectors carrying SIGLEC15 shRNA may be transfected into lymph node single cell obtained as above using a transfection kit, e.g. from Shanghai GenePharma Company (Shanghai, China), according to the manufacturer's instructions. After forty-eight hours incubation, knockdown of *SIGLEC15* expression is confirmed by RT-PCR. Other protocols as well as reagents for downregulating expression of a specific gene by shRNA are also commercially available from a number of companies, including without prejudice ThermoFisher Scientific, and Sigma-Aldrich.

In some embodiments, the expression of *SIGLEC15* is reduced by downregulation of expression of a gene encoding SIGLEC15 by CRISPR-mediated gene editing. CRISPR/Cas9 vectors are constructed for targeting the selected specific sites and regions within the *SIGLEC15* gene. sgRNAs are designed using CR!SPRdirect (http://crispr.dbcls.jp/). The sgRNA oligomers are synthesized and cloned into the pU6gRNACas9EGFP vector. Lymph node single cells are first seeded in 6-well plates and then transfected using Lipofectamine 2000 (ThermoFisher Scientific) following the manufacturer's instructions. After the cells are incubated for an additional 48 hours, knockout of *SIGLEC15* expression was confirmed by RT-PCR and flow cytometry.

After having reduced expression of *SIGLEC15,* the extracted cells are cultured *ex vivo* under conditions favouring T cell expansion. Such protocols and reagents therefor are known *per se,* and are available i.a. under the trade names Gibco^{™} CTS^{™} OpTmizer^{™}, CTS AIM V medium,and CTS Immune Cell SR (ThermoFisher Scientific), and from Stem Cell Technologies Inc. (Cambridge, MA, USA) as disclosed in Technical Bulletin #27143). Protocols for expansion of T cells is also disclosed in WO2018234516 and Chinese patent application CN108220234.

Generally, single cells with reduced expression of *SIGLEC15* suspensions are re-suspended in a serum-free cell culture medium in the presence of interleukin-2 and/or other cytokines, and transferred to a growth container such as a flask or plate. The cells are then expanded in a 5 % CO₂-rich atmosphere at 37 °C and re-stimulated by the tumor antigens together with antigen presenting cells during cell cultures.

The following protocol (8) is provided as one possible protocol. Single-cell suspensions obtained from SLNs are resuspended in X-VIVO^{™} 15 serum-free cell culture medium (LONZA) at a density of 4 × 10⁶ cells/ml in the presence of 1000 IU/ml recombinant human interleukin-2 (Shuanglu, China). These cells are plated in flasks or plates and maintained in a humidified atmosphere containing 5 % CO₂ at 37 °C. The autologous tumor lysate is added to the initial culture at a dilution of 1/100 (v/v) as described previously (1). To induce highly tumor-specific SLN-T cells, re-stimulation is performed by adding autologous tumor lysate together with irradiated autologous PBMCs during SLN-T cell cultures. One week before transfusion, 5 ml of culture medium is removed for a bacterial and fungal contamination test using BACTEC 9120 (Becton-Dickinson), and the endotoxin levels are measured based on the Limulus reaction. On the day of transfusion, these assays are repeated to detect any bacterial, fungal or endotoxin contamination. The lymphocyte subsets of SLN-T cells are analyzed.

Furthermore, 1 × 10⁶ cells were used for flow cytometry analysis of the tumor surface marker epithelial cell adhesion molecule (EpCAM) to exclude the presence of tumor cells.

In a further aspect, the present invention relates to a population of T cells with reduced expression of *SIGLEC15* obtained or obtainable by the method according to the aspects above.

In a further aspect, the present invention relates to a population of T cells with reduced expression of *SIGLEC15* obtained or obtainable by the method according to the invention for use in medicine.

In one embodiment of this aspect, the invention relates to a population of T cells with reduced expression of *SIGLEC15* obtained or obtainable by the method according to the aspects above for use in the treatment of cancer, such as the treatment of solid tumors.

In one embodiment, the invention relates to the use of autologous T cells in a method for treatment of a cancerous tumor. According to this embodiment, a population of T cells obtained or obtainable by a method according to the aspects above may be used in treatment of the cancerous tumor located in proximity to the lymph node identified as a tumor-draining lymph node according to the methods discussed above, in the subject from which the tumor-draining lymph node is obtained.

The cancerous tumor may be any form of solid cancer. A solid cancer according to the present invention is an abnormal mass of tissue that originates in an organ. A solid cancer usually does not contain cysts or liquid areas. The solid cancer may be malignant. Different types of solid cancers are named for the type of cells that form them. Types of solid cancer include sarcomas, carcinomas, and lymphomas. The present invention provides a composition comprising T-cells obtained of obtainable by the method of the invention.

Examples of solid cancers include adrenal cancer, anal cancer, anaplastic large cell lymphoma, angioimmunoblastic T-cell lymphoma, B-cell lymphoma, bile duct cancer, urinary bladder cancer, brain/CNS tumors, breast cancer, cervical cancer, colon cancer, endometrial cancer, esophagus cancer, ewing family of tumors, eye cancer, gallbladder cancer, gastrointestinal carcinoid tumors, gastrointestinal stromal tumor (gist), gestational trophoblastic disease, hepatosplenic T-cell lymphoma, Hodgkin's lymphoma, intravascular large B-cell lymphoma, kidney cancer, laryngeal and hypopharyngeal cancer, liver cancer, lung cancer (non-small cell and small cell), lung carcinoid tumor lymphomatoid granulomatosis, malignant mesothelioma, nasal cavity and paranasal sinus cancer, nasopharyngeal cancer, neuroblastoma, nodal marginal zone B cell lymphoma, non- Hodgkin's lymphoma, oral cavity and oropharyngeal cancer, osteosarcoma, ovarian cancer, pancreatic cancer, penile cancer, pituitary tumors, primary effusion lymphoma, prostate cancer, retinoblastoma, rhabdomyosarcoma, salivary gland cancer, sarcoma, skin cancer (basal and squamous cell, melanoma and merkel cell), small intestine cancer, stomach cancer, testicular cancer, thymus cancer, thyroid cancer, uterine sarcoma, vaginal cancer, vulvar cancer, Waldenstrom macroglobulinemia, and Wilms' tumor. The T-cells of the invention are especially effective in the treatment of solid cancers. As such, the subject to be treated with the therapeutic method of the invention may have a solid cancer. The T-cells of the invention are particularly effective in the treatment of solid cancers selected from the group consisting of: anal cancer, urinary bladder cancer, breast cancer, cervical cancer, colon cancer, liver cancer, lung cancer (non- small cell and small cell), lung carcinoid tumor, ovarian cancer, pancreatic cancer, penile cancer, prostate cancer, stomach cancer, testicular cancer, uterine sarcoma, vaginal cancer, vulvar cancer, and even more especially for the treatment of breast cancer, colon cancer, liver cancer, lung cancer (non-small cell and small cell), lung carcinoid tumor, pancreatic cancer, prostate cancer, ovarian cancer and urinary bladder cancer.

In one embodiment, the cancer is selected from the group of colorectal cancer, malignant melanoma, cervical carcinoma, Head & Neck Squamous Cell Carcinoma (HNSCC), Non-Small Cell Lung Carcinoma (NSCLC).

Also disclosed herein is a method, not being part of the claimed invention, of treatment of a cancerous tumor in a subject, said method comprising
S1. Identifying a lymph node draining lymphatic fluid from the cancerous tumor said subject;
S2. Extracting cells from the identified lymph node;
S3. Reducing expression of *SIGLEC15* in the extracted cells;
S4. Expanding the extracted cells with reduced expression of *SIGLEC15* under conditions favouring T cell expansion; and
S5. Administering the expanded T cells with reduced expression of *SIGLEC15.*

Step S1 can be exchanged for step S1' as discussed above.

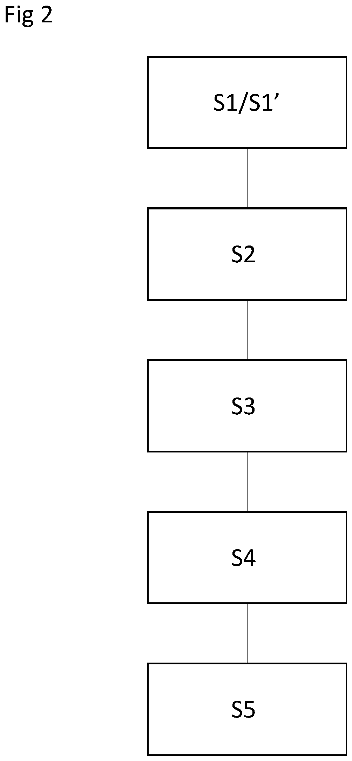

The method of treatment according to the disclosure is schematically illustrated in Figure 2.

Steps S1, S2, S3 and S4 can be performed as described above.

Administration of the expanded T cells may be done by intravenous administration as known in the art (11). Administration may also be intraarterial, intrathecal or intraperitoneal.

The following protocol (8) is provided as one useful protocol for administration of T cells. The final SLN-T cells are harvested, washed twice in saline solution and transferred to a sterile plastic bag containing 200 ml of saline solution and 1 % human serum albumin (CSL Behring GmbH, Germany). The cells are intravenously transfused over a 60-min interval according to the blood transfusion guidelines of the hospital. Transfusion-related toxicity is assessed post-cell transfusion using the Common Terminology Criteria for Adverse Events (CTCAE) 3.0 criteria.

Also disclosed herein is the use of a population of T cells obtained or obtainable according to the methods described above in the manufacture of a pharmaceutical composition. In one embodiment, the pharmaceutical composition is for use in the treatment of cancer, such as the treatment of solid tumors.

The present invention also relates to a pharmaceutical composition comprising a population of T cells obtained or obtainable according to the methods described above. In one embodiment, the pharmaceutical composition is for use in medicine as described above, such as in the treatment of cancer, such as the treatment of solid tumors.

Said pharmaceutical compositions may comprise pharmaceutically acceptable excipients as is common in the art. The pharmaceutical compositions are preferably formulated in liquid for suitable for injection, such as intravenous intraarterial, intrathecal or intraperitoneal administration.

A "pharmaceutical excipient" or a "pharmaceutically acceptable excipient" is a carrier, usually a liquid, in which an active therapeutic agent is formulated. In one embodiment of the invention, the active therapeutic agent is a population of T cells obtained or obtainable by the methods according to the invention. The excipient generally does not provide any pharmacological activity to the formulation, though it may provide chemical and/or biological stability. Exemplary formulations can be found, for example, in Remington's Pharmaceutical Sciences, 19th Ed., Grennaro, A., Ed., 1995.

As used herein "pharmaceutically acceptable carrier" or "excipient" includes any and all solvents, dispersion media, coatings, antibacterial and antifungal agents, isotonic and absorption delaying agents that are physiologically compatible. In one embodiment, the carrier is suitable for parenteral administration. Alternatively, the carrier can be suitable for intravenous, intraperitoneal, intramuscular, or sublingual administration. Pharmaceutically acceptable carriers include sterile aqueous solutions or dispersions for the extemporaneous preparation of sterile injectable solutions or dispersions. The use of such media and agents for pharmaceutically active substances is well known in the art. Except insofar as any conventional media or agent is incompatible with the active compound, use thereof in the pharmaceutical compositions of the invention is contemplated. Supplementary active compounds can also be incorporated into the compositions.

In performing the present invention, the skilled person may use methods and protocols as known in the art, including but not exclusively as disclosed in patent documents and scientific articles referenced herein, as well as references in such documents.

### Experimental

### Gene expression analysis

Sentinel and non-sentinel lymph nodes were identified as previously described (7). In brief, a lymphotropic dye (patent blue, Sigma-Aldrich) was injected under the serosa surrounding the primary tumour. Tumor-draining lymph nodes, i.e. sentinel nodes, were stained blue. Lymph nodes not stained were considered non-sentinel nodes.

Two lymph nodes, one non-metastatic sentinel lymph node (SN) and one non-sentinel lymph nodes(NSN) lymph node, were obtained from each of twenty-three patients diagnosed with colorectal cancer, and used to get the gene expression profiles by high-throughput RNA Sequencing technology and bioinformatics analysis.

Gene expression data was obtained by sequencing lymph node tissues using Ilumina's RNA-SEQ technique.

A total of 16 genes including 9 up- and 7 downregulated genes were differentially expressed in sentinel lymph nodes compared with non-sentinel lymph nodes. *IL1RL1, STON2, TPSAB1, GATA2, DNAJB4, NR2F1,* and *DIPK2A* were found to be downregulated and *PLA2G2D, ZBED6CL, SIGLEC15, KCNC3, ATP2A1, MMP2-AS1, FBXO41, DSC2,* and *TFEC* were found to be up-regulated.

### Validation via RT-PCR

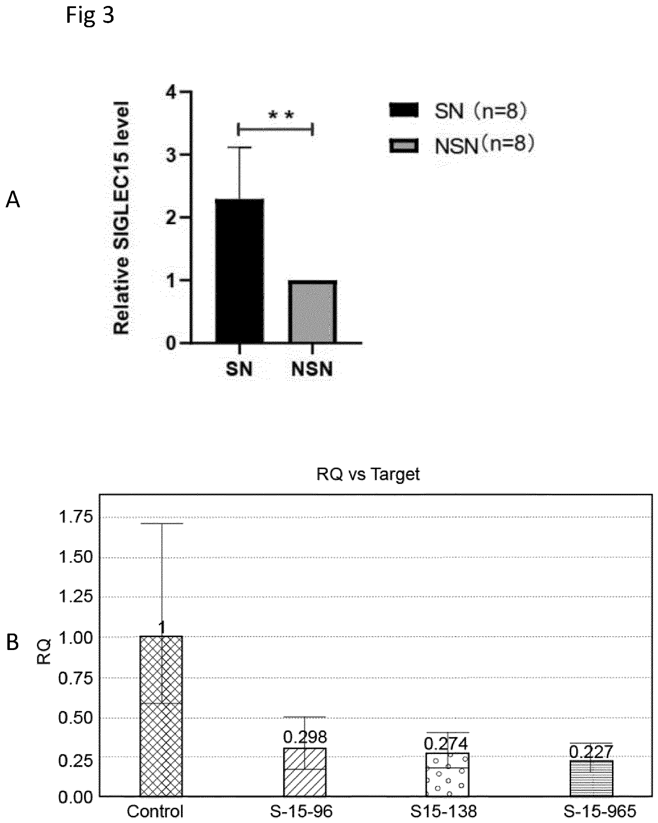

Total RNA was extracted from pairs of SN and NSN from eight patients randomly selected from the above 23 patients, using TRIzol reagent following the manufacturer's instructions. cDNA synthesis was performed using the PrimeScript^{™} RT reagent Kit with gDNA Eraser (Takara , Japan). The primers of *SIGLEC15* and endogenous reference gene β-actin were designed using Primer5 software. TB Green^{®} Premix Ex Taq ^{™} II (Takara , Japan) was used to perform RT-qPCR following the manufacturer's instructions. Melt curve analysis was carried out after the PCR to confirm primer specificity, and the relative level of *SIGLEC15* was calculated using 2-ΔΔCt method (12). Relative expression of *SIGLEC15* in sentinel lymph nodes before and after siRNA knock-down was analysed in three sentinel lymph nodes. Results are shown in Tables 1 and 2 and Figure 3.

**Table 1: Relative expression of SIGLEC15 in sentinel and non-sentinel lymph nodes, respectively.**

| **Sample** | **SN** | **NSN** |
|---|---|---|
| Sample 1 | 2.133172171 | 1 |
| Sample 2 | 1.277509861 | 1 |
| Sample 3 | 2.716344768 | 1 |
| Sample 4 | 3.626724246 | 1 |
| Sample 5 | 1.819656602 | 1 |
| Sample 6 | 3.040552625 | 1 |
| Sample 7 | 2.413287155 | 1 |
| Sample 8 | 1.318898005 | 1 |
| *Mean* | *2.293268179* | *1* |
| *SD* | *0.824711061* | *0* |

**Table 2: Relative expression of SIGLEC15 in sentinel lymph nodes before and after siRNA knock-down.**

| | **Control** | **S15-96** | **S15-965** | **S15-138** |
|---|---|---|---|---|
| **SIGLEC15 (Ct mean)** | 31.978 | 31.071 | 31.262 | 31.58 |
| ***β-ACTIN* (Ct mean)** | 18.807 | 16.155 | 15.953 | 16.54 |
| **Δct** | 13.171 | 14.916 | 15.309 | 15.04 |
| **ΔΔct** | 0 | 1.745 | 2.138 | 1.869 |
| **2-ΔΔct** | 1 | 0.298 | 0.227 | 0.274 |

### RNAscope in situ hybridization

To further explore the expression pattern of *SIGLEC15* in lymph nodes, we investigated the expression of *SIGLEC15* and CD163 in pairs of SN and NSN from four patients using RNAscope *in situ* hybridization technology (13), which can provide information on the spatial expression of RNA in tissue cells. The results are disclosed in Table 3 and show that mRNA expression is higher in sentinel lymph nodes (p=0.042, Student's t-test).

**Table 3: expressed SIGLEC15 mRNA analysed by RNAscope.**

| | SN | NSN |
|---|---|---|
| Sample1 | 70.65 | 61.58 |
| Sample2 | 69.57 | 49.61 |
| Sample3 | 60.87 | 56.99 |
| Sample4 | 59.83 | 44.70 |
| Mean | 65.23 | 53,22 |

According to the double staining results of *SIGLEC15* and CD163 in the sentinel and non-sentinel lymph nodes slices, we can further confirm that *SIGLEC15* is highly expressed in SN compared with NSN. In addition, the *SIGLEC15* was not only expressed in M2 macrophage but also in other cell type. From this result, we can further confirm that *SIGLEC15* is highly expressed in SN compared with NSN, *SIGLEC15* was not only expressed in M2 macrophage but also expressed in other cells.

### Flow cytometry analysis

We detected SIGLEC15 protein expression on the surface of different cell subset by flowcytometry.

Single-cell suspensions from SNs or NSNs and tumor tissue were obtained immediately after surgery by applying gentle pressure using a loose-fit glass homogenizer.

For SIGLEC15 analysis of the cells from SNs or NSNs , fluorescent-labeled monoclonal antibodies (mAbs) againstCD45, CD86, CD11c, CD163, CD11b, CD15, CD14, CD3, CD4, CD8, CD16, CD56, CD19, and SIGLEC15 (Biolegend) were used. Cells were incubated in the presence of mAbs according to the manufacturer's recommendations for 20 min at room temperature (18-25 °C) and protected from light. After incubation, the cell suspensions were washed with phosphate-buffered saline (PBS), and the cell pellets were resuspended in 0.5 ml of PBS for analysis. Samples were further analyzed using a Navios flow cytometer (Beckman Coulter). At least 50,000 total events were collected and analyzed using Flowjo software (Flowjo LCC).

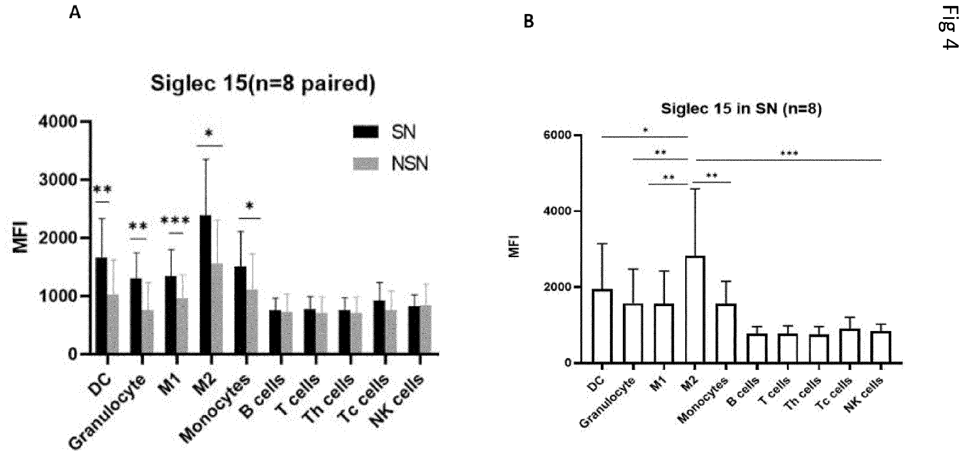

SIGLEC15 was relatively highly expressed in almost all subgroups of sentinel lymph node (Figure 4A). For sentinel lymph nodes, SIGLEC15 was relatively highly expressed in M2 macrophage (Figure 4B), and the same trend was observed for non-sentinel lymph nodes (data not shown).

### SIGLEC15 function analysis

To evaluate the role of SIGLEC15 in sentinel lymph nodes, we knocked down the expression of *SIGLEC15* through siRNA technology in sentinel lymph nodes. Briefly, transfection of lymph node single cell with siRNA was performed according to the manufacturer's instruction (GE Dharmacon).

In order to confirm the knockout effect, we synthesized a total of 3 siRNA sequences. siRNA were designed according to the RNA sequence of targeted gene (*SIGLEC15*)*,* and using siRNA design software (Invivogen, https://www.invivogen.com/sirnawizard/guidelines.php) following the siRNA design principles.

The following siRNA sequences were designed (incl. complementary strands and 3'-TT overhang to prevent degradation by 3'-exonucleases):

**Table 4: siRNA sequences**

| | Sequence | SEQ ID NO | Complementary sequence | SEQ ID NO |
|---|---|---|---|---|
| S15-965 | UCUCCCGACAGGCUCAUUUTT | 1 | AAAUGAGCCUGUCGGGAGATT | 2 |
| S15-138 | GGAGAACUUGCUCAACACATT | 3 | UGUGUUGAGCAAGUUCUCCTT | 4 |
| S15-96 | AGGCCCAGGAGUCCAAUUATT | 5 | UAAUUGGACUCCUGGGCCUTT | 6 |

1 µmol/L siRNA was transfected in a 96-well tissue culture plate with Accell siRNA delivery media for 72 hours. RT-PCR was used to assess *SIGLEC15* mRNA expression in the siRNA transfected and non-transfected groups.

Flow cytometry was used to detect the mean fluorescence intensity (MFI) of SIGLEC15 protein on the surface of all living cells in lymph nodes to evaluate the effect of siRNA interference on the reduction of SIGLEC15 protein expression. Results are shown in Table 5.

**Table 5: Mean Fluorescence Intensity (MFI) of SIGLEC15 expression on live lymph node cells**

| | **control** | **S15-96** | **S15-965** | **S15-138** |
|---|---|---|---|---|
| sample1 | 917 | 812 | 780 | 746 |
| sample2 | 1026 | 999 | 800 | 688 |
| samples | 999 | 804 | 759 | 598 |
| sample4 | 1536 | 1240 | 1161 | 1041 |
| sample5 | 1327 | 924 | 1046 | 915 |
| Mean | 1161 | 955.8 | 909.2 | 797.6 |
| SD | 260.9 | 178.4 | 182.5 | 178.6 |

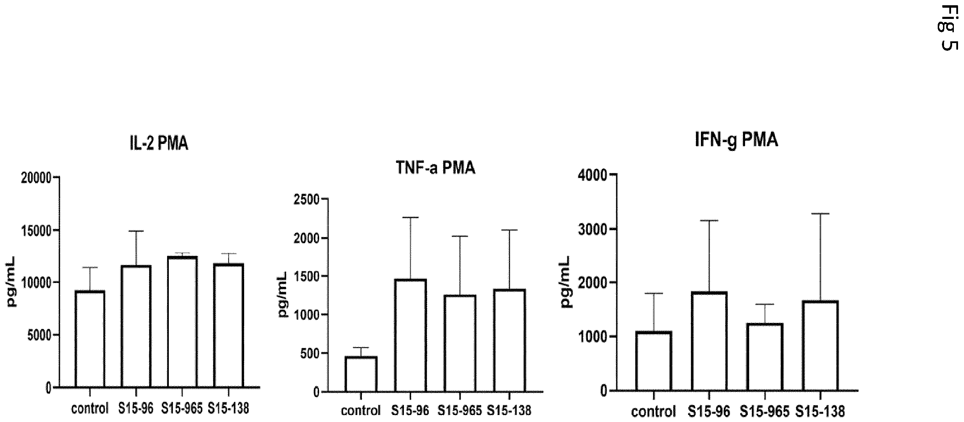

Flow cytometry data showed that the anti-tumor functional cytokines released by T cells, such as IL-2, TNFα and IFNγ in cells from sentinel lymph nodes were up-regulated after SIGLEC15 knockdown. (Table 6 and Figure 5).

**Table 6: T cell functional cytokine release after SIGLEC15 knock-down**

| **IL-2(pg/mL)** | **control** | **S15-96** | **S15-965** | **S15-138** |
|---|---|---|---|---|
| **sample 1** | 7010 | 7987 | 12168 | 10778 |
| **sample 2** | 9463 | 12956 | 12485 | 12219 |
| **sample 3** | 11308 | 14097 | 12839 | 12467 |
| ***Average*** | ***9260*** | ***11680*** | ***12497*** | ***11821*** |
| | | | | |

| **TNF-α (pg/mL)** | **control** | **S15-96** | **S15-965** | **S15-138** |
|---|---|---|---|---|
| **sample 1** | 564 | 565 | 688 | 722 |
| **sample 2** | 477 | 1775 | 965 | 2194 |
| **sample 3** | 348 | 2065 | 2129 | 1091 |
| ***Average*** | ***463*** | ***1468*** | ***1261*** | ***1336*** |
| | | | | |

| **IFN-γ(pg/mL)** | **control** | **S15-96** | **S15-965** | **S15-138** |
|---|---|---|---|---|
| **sample 1** | 968 | 613 | 852 | 881 |
| **sample 2** | 1857 | 3239 | 1456 | 3517 |
| **sample 3** | 473 | 1663 | 1446 | 622 |
| ***Average*** | ***1099*** | ***1838*** | ***1251*** | ***1673*** |

After *SIGLEC15* blocking, the functional T cell cytokines were increased, which is recognized as enhanced anti-tumor effect of T cells.

IL2 was mainly secreted by Th1 T cell. IL2 is the marker of activated Th cells and cytotoxic T cells and NK cells, it is also the necessary element for the proliferation of activated T cells.

TNFα is mainly produced by activated T lymphocytes, and natural killer (NK) cells. It induces hemorrhagic necrosis in a certain set of tumor types, and is used in regional treatment of locally advanced soft tissue sarcomas and metastatic melanomas. TNFα also causes an inflammatory response, which induces more immune cells to kill the tumor.

IFNy plays a key role in activation of cellular immunity and subsequently, stimulation of antitumor immune-response. It acts as a cytotoxic cytokine together with granzyme B and perforin to initiate apoptosis in tumor cells, but also enables the synthesis of immune checkpoint inhibitory molecules and indoleamine-2,3-dioxygenase (IDO), thus stimulating other immune-suppressive mechanisms.

Consequently, the cytokine release profile is indicative of an anti-tumor effect of the expanded T cells with reduced expression of *SIGLEC15.*

### References

1. Pilot Study of Sentinel-Node-Based Adoptive Immunotherapy in Advanced Colorectal Cancer. Karlsson, M., et al. 2010, Ann Surg Oncol, Vol. 17, pp. 1747-1757.
*2.* Generation of tumor-infiltrating lymphocyte cultures for use in adoptive transfer therapy for melanoma patients. Dudley, M.E., et al.,. 4, 2003, Journal of Immunotherapy, Vol. 26, pp. 332-342.
3. A guide to cancer immunotherapy: from T cell basic science to clinical practice. Waldman, A.D., et al. 2020, Nature Reviews Immunology, Vol. 20, pp. 651-668.
*4.* SIGLEC15: an immune system Siglec conserved throughout vertebrate evolution. Angata, T., et al. 8, 2007, Glycobiology, Vol. 17, pp. 838-846.
*5.* SIGLEC15 as an immune-suppressor and potential target for normalization cancer immunotherapy. Wang, J. et al. 4, 2019, Nature Medicine, Vol. 25, pp. 656-666.
*6.* Optimizing CAR-T Cell Manufacturing Process during Pivotal Clinical Trials. Tyagarajan, S., et al. March 2020, Molecular Therapy: Methods & Clinical Development, Vol. 16, pp. 136-144.
*7.* Identification of sentinel nodes in patients with colon cancer. Dahl, K., et al. 4, May 2005, Eur J Surg Oncol, Vol. 31, pp. 381-385.
*8.* Phase I/II study of adjuvant immunotherapy with sentinel lymph node T lymphocytes in patients with colorectal cancer. Zhen, Y-H, et al. s.l. : Springer, 20 May 2015, Cancer Immunol Immunother.
9. RNA Interference to Knock Down Gene Expression. Han, H. 2018, Methods Mol Biol, Vol. 1706, pp. 293-302.
10. Short Hairpin RNA (shRNA): Design, Delivery, and Assessment. Moore, C.B., et al. 2010, Methods Mol Biol. , Vol. 629, pp. 141-158.
11. Tumor-infiltrating lymphocytes for the treatment of metastatic cancer. Foppen, M.H.G. et al.,. 2015, MOLECULAR ON COLOGY, Vol. 9, pp. 1918-1935.
12. Analysis of Relative Gene Expression Data Using Real-Time Quantitative PCR and the 2-ΔΔCT Method. Livak, K.J., et al. 4, December 2001, Methods, Vol. 25, pp. 402-408.
13. RNAscope: A novel in situ RNA analysis platform for formalin -fixed, Paraffin-embedded tissues. Wang, F., et al. 1, 2012, The Journal of Molecular Diagnostics, Vol. 14, pp. 22-29.

## Claims

1. A method for obtaining a population of T-cells having reduced expression of *SIGLEC15,* comprising
S1'. Providing a T cell containing lymph node tissue removed from a subject, said lymph node tissue being obtained from a lymph node identified as a lymph node draining lymphatic fluid from a cancerous tumor in said subject;
S2. Extracting cells from the identified lymph node;
S3. Reducing expression of *SIGLEC15* in the extracted cells; and
S4. Expanding the extracted cells with reduced expression of *SIGLEC15* under conditions favouring T cell expansion,
wherein the expression of *SIGLEC15* is reduced by downregulation of expression of a gene encoding SIGLEC15 by siRNA transfection, shRNA transfection, or CRISPR-mediated gene editing.

2. The method according to claim 1, wherein the expression of *SIGLEC15* is reduced by downregulation of expression of a gene encoding SIGLEC15 by siRNA transfection using an siRNA molecule having a nucleotide sequence according to SEQ ID NO: 1, SEQ ID NO: 2, SEQ ID NO: 3, SEQ ID NO: 4, SEQ ID NO: 5, or SEQ ID NO: 6.

3. The method according to any one of claims 1-2, wherein the conditions favouring T cell expansion comprise maintenance of the cells in the presence of interleukin-2.

4. A population of T cells with reduced expression of *SIGLEC15* obtainable by the method according to any one of claims 1-3, for use in medicine.

5. The population of T cells for use according to claim 4, wherein the use is in a method for treatment of a cancer.

6. The population of T cells for use according to claim 5 wherein the cancer is a solid tumor.

7. The population of T cells for use according to claim 5 or 6, wherein the cancer is the cancerous tumor of the subject from which the T cells originates, or a metastasis thereof.

8. The population of T cells for use according to any one of claims 5-7, wherein the cancer is selected from the group of colorectal cancer, malignant melanoma, cervical carcinoma, Head & Neck Squamous Cell Carcinoma (HNSCC), Non-Small Cell Lung Carcinoma (NSCLC).

9. A pharmaceutical composition comprising a population of T cells with reduced expression of *SIGLEC15* obtainable by the method according to any one of claims 1-3, and optionally pharmaceutically acceptable excipients and or carriers.

## Patentansprüche

1. Verfahren zum Erlangen einer Population von T-Zellen mit einer reduzierten Expression von *SIGLEC15,* umfassend
S1. Bereitstellen einer T-Zelle, die Lymphknotengewebe enthält, das aus einem Subjekt entnommen wird, wobei das Lymphknotengewebe aus einem Lymphknoten erlangt wird, der als ein Lymphknoten identifiziert ist, der Lymphflüssigkeit aus einem Krebstumor in dem Subjekt drainiert;
S2. Extrahieren von Zellen aus dem identifizierten Lymphknoten; S3. Reduzieren der Expression von *SIGLEC15* in den extrahierten Zellen; und
S4. Expandieren der extrahierten Zellen mit der reduzierten Expression von *SIGLEC15* unter Bedingungen, welche die T-Zell-Expansion begünstigen,
wobei die Expression von *SIGLEC15* durch eine Herabregulierung der Expression eines Gens, das für SIGLEC15 codiert, durch siRNA-Transfektion, shRNA-Transfektion oder CRISPR-vermittelte Genom-Editierung reduziert wird.

2. Verfahren nach Anspruch 1, wobei die Expression von *SIGLEC15* durch die Herabregulierung der Expression eines Gens, das für SIGLEC15 codiert, durch siRNA-Transfektion unter Verwendung eines siRNA-Moleküls mit einer Nukleotidsequenz gemäß SEQ ID NO: 1, SEQ ID NO: 2, SEQ ID NO: 3, SEQ ID NO: 4, SEQ ID NO: 5 oder SEQ ID NO: 6 reduziert wird.

3. Verfahren nach einem der Ansprüche 1-2, wobei die Bedingungen, welche die T-Zell-Expansion begünstigen, die Haltung der Zellen in der Gegenwart von Interleukin-2 umfassen.

4. Population von T-Zellen mit reduzierter Expression von *SIGLEC15,* die durch das Verfahren nach einem der Ansprüche 1-3 erlangbar ist, zur Verwendung in der Medizin.

5. Population von T-Zellen zur Verwendung nach Anspruch 4, wobei die Verwendung in einem Verfahren zur Behandlung einer Krebserkrankung erfolgt.

6. Population von T-Zellen zur Verwendung nach Anspruch 5, wobei die Krebserkrankung ein solider Tumor ist.

7. Population von T-Zellen zur Verwendung nach Anspruch 5 oder 6, wobei die Krebserkrankung ein Krebstumor des Subjekts, aus dem die T-Zellen stammen, oder eine Metastase davon ist.

8. Population von T-Zellen zur Verwendung nach einem der Ansprüche 5-7, wobei die Krebserkrankung ausgewählt ist aus der Gruppe von Kolorektalkrebs, malignem Melanom, Zervixkarzinom, Kopf-Hals-Plattenepithelkarzinom (HNSCC), nichtkleinzelligem Lungenkarzinom (NSCLC).

9. Pharmazeutische Zusammensetzung, umfassend eine Population von T-Zellen mit reduzierter Expression von *SIGLEC15,* die durch das Verfahren nach einem der Ansprüche 1-3 erlangbar ist, und optional pharmazeutisch unbedenkliche Hilfsstoffe und/oder Träger.

## Revendications

1. Procédé pour obtenir une population de lymphocytes T ayant une expression réduite de *SIGLEC15,* comprenant
S1'. La fourniture d'un tissu de ganglion lymphatique contenant des lymphocytes T prélevés sur un sujet, ledit tissu de ganglion lymphatique étant obtenu à partir d'un ganglion lymphatique identifié comme un ganglion lymphatique drainant le fluide lymphatique d'une tumeur cancéreuse chez ledit sujet ;
S2. L'extraction de cellules du ganglion lymphatique identifié ;
S3. La réduction de l'expression de *SIGLEC15* dans les cellules extraites ; et
S4. L'expansion des cellules extraites avec une expression réduite de *SIGLEC15* dans des conditions favorisant l'expansion de lymphocytes T,
dans lequel l'expression de *SIGLEC15* est réduite par la régulation à la baisse de l'expression d'un gène codant SIGLEC15 par transfection d'ARNsi, transfection d'ARNsh ou édition de gène médiée par CRISPR.

2. Procédé selon la revendication 1, dans lequel l'expression de *SIGLEC15* est réduite par régulation à la baisse de l'expression d'un gène codant SIGLEC15 par transfection d'ARNsi à l'aide d'une molécule d'ARNsi ayant une séquence nucléotidique selon SEQ ID NO : 1, SEQ ID NO : 2, SEQ ID NO : 3, SEQ ID NO : 4, SEQ ID NO : 5 ou SEQ ID NO : 6.

3. Procédé selon l'une quelconque des revendications 1 à 2, dans lequel les conditions favorisant l'expansion de lymphocytes T comprennent le maintien des cellules en présence d'interleukine-2.

4. Population de lymphocytes T avec une expression réduite de *SIGLEC15* pouvant être obtenue par le procédé selon l'une quelconque des revendications 1 à 3, pour une utilisation en médecine.

5. Population de lymphocytes T pour une utilisation selon la revendication 4, dans laquelle l'utilisation est dans un procédé de traitement d'un cancer.

6. Population de lymphocytes T pour une utilisation selon la revendication 5, dans laquelle le cancer est une tumeur solide.

7. Population de lymphocytes T pour une utilisation selon la revendication 5 ou 6, dans laquelle le cancer est la tumeur cancéreuse du sujet dont proviennent les lymphocytes T, ou une métastase de celle-ci.

8. Population de lymphocytes T pour une utilisation selon l'une quelconque des revendications 5 à 7, dans laquelle le cancer est choisi dans le groupe du cancer colorectal, du mélanome malin, du carcinome cervical, du carcinome épidermoïde de la tête et du cou (HNSCC), du carcinome du poumon non à petites cellules (NSCLC).

9. Composition pharmaceutique comprenant une population de lymphocytes T avec une expression réduite de *SIGLEC15* pouvant être obtenue par le procédé selon l'une quelconque des revendications 1 à 3, et éventuellement des excipients et/ou supports pharmaceutiquement acceptables.
